# EUROPEAN PATENT SPECIFICATION

(11) **EP 2 468 311 B1**
(45) Date of publication and mention of the grant of the patent: **21.10.2015**
(21) Application number: 10809983.9
(22) Date of filing: 18.08.2010
(51) Int. Cl.: A61L 27/00, A61F 2/34

(54) **HIGHLY LUBRICATING SLIDING MEMBER AND ARTIFICIAL JOINT USING SAME**
IN HOHEM MASSE SCHMIERFÄHIGES GLEITELEMENT UND KÜNSTLICHES GELENK DAMIT
ÉLÉMENT COULISSANT HAUTEMENT LUBRIFIÉ ET PROTHÈSE ARTICULAIRE UTILISANT CELUI-CI

(30) Priority: 20.08.2009 JP 2009190852
(43) Date of publication of application: 27.06.2012
(73) Proprietor: Kyocera Medical Corporation, Osaka-shi, Osaka 532-0003 (JP); The University of Tokyo, Bunkyo-ku, Tokyo 113-8654 (JP)
(72) Inventor: KYOMOTO, Masayuki, Osaka-shi Osaka 532-0003 (JP); ISHIHARA, Kazuhiko, Tokyo 113-8654 (JP)
(74) Representative: Hoffmann Eitle
(86) International application number: PCT/JP2010/063929
(87) International publication number: WO 2011/021642

(56) References cited:
- EP-A1- 1 433 488
- EP-A1- 1 987 849
- WO-A1-2010/058848
- WO-A1-2010/058848
- WO-A1-2010/074238
- WO-A1-2010/074238
- JP-A- 61 013 963
- JP-A- 2007 202 965
- JP-A- 2008 054 788
- JP-A- 2009 528 110
- MASAYUKI KYOMOTO ET AL: "Self-initiated surface grafting with poly(2-methacryloyloxyethyl phosphorylcholine) on poly(ether-ether-ketone)", BIOMATERIALS, ELSEVIER SCIENCE PUBLISHERS BV., BARKING, GB, vol. 31, no. 6, 10 November 2009 (2009-11-10), pages 1017-1024, XP008154073, ISSN: 0142-9612, DOI: 10.1016/J.BIOMATERIALS.2009.10.055 [retrieved on 2009-11-10]
- MASAYUKI KYOMOTO ET AL: "Self-initiated surface graft polymerization of 2-methacryloyloxyethyl phosphorylcholine on poly(ether ether ketone) by photoirradiation", ACS APPLIED MATERIALS,, vol. 1, no. 3, 16 February 2009 (2009-02-16), pages 537-542, XP008154069, DOI: 10.1021/AM800260
- KYOMOTO M. ET AL: 'Self-Initiated Surface Graft Polymerization of 2-Methacryloyloxyethyl Phosphorylcholine on Poly(ether ether ketone) by Photoirradiation' ACS APPLIED MATERIALS vol. 1, no. 3, 2009, pages 537 - 542, XP008154069
- E.P.J. WATTERS ET AL: 'Wear of artificial hip joint material' CHEMICAL ENGINEERING JOURNAL vol. 112, no. 1-3, 01 September 2005, pages 137 - 144, XP008154070
- KYOMOTO M. ET AL: 'Self-initiated surface grafting with poly(2-methacryloyloxyethyl phosphorylcholine) on poly(ether-ether-ketone)' BIOMATERIALS vol. 31, no. 6, 2010, pages 1017 - 1024, XP008154073
- MASAYUKI KYOMOTO ET AL.: 'Poly Hokozoku Ketone Hyomen kara no Jiko Kaishi Hikari Graft Jugo ni yoru Seitai Shinwasei Polymer-so no Kochiku, 2C21' DAI 31 KAI THE ANNUAL MEETING OF THE JAPANESE SOCIETY FOR BIOMATERIALS YOKOSHU 16 November 2009, page 182, XP008154736

## Description

### TECHNICAL FIELD

The present invention relates to a medical material and a method for producing the same, and particularly relates to a sliding material to be used for an artificial joint that restores human joints and a method for producing the same. The present invention also relates to a sliding material which can maintain a slidability state of slidable parts for a long period of time, and particularly relates to a polymer material for an artificial joint and the artificial joint formed by said polymer material.

### BACKGROUND OF THE INVENTION

In general, polyethylene (mainly ultrahigh molecular weight polyethylene, hereinafter referred to as PE) has been conventionally used as a component of artificial joints, such as an artificial hip joint and an artificial knee joint. However, when the artificial joint was used in vivo, there was a tendency that lysis of bone (i.e. osteolysis) had been induced by wear debris of PE which was produced through a frictional movement. When the osteolysis occurs, so-called loosening in which the fixing force of an artificial joint and a bone becomes weaker arises, and the loosening had become a big problem as complication of an arthroplasty. Usual wear of the above-mentioned PE was about 0.1 to 0.2 mm per year, and there was no problem for a certain period of time (for example, for about several years) after the arthroplasty. However, the amount of the above-mentioned loosening became remarkable after a lapse of about five years, and thus a re-operation of exchanging the artificial joint should be needed, and that could impose a heavy burden on the patients.

In the artificial hip joint, the size of a femoral head component has been enlarged for the purpose of improving the range of motion and of prevention of dislocation. Since there is a limit for the size of a cup to be housed in an acetabulum, thinning of (the thickness of) the acetabular cup formed by PE has been required corresponding to the enlarging the size of the femoral head component. However, there was a limit in advancing the thinning of the acetabular cup due to the viewpoints of the properties of wear resistance, deformation resistance and fracture resistance.

Intensive researches of alternate material of PE to be used for an artificial joint have also been carried out; thereby polyetheretherketone (hereinafter referred to as PEEK) is taken as an example thereof, which is an engineering plastic excellent in the properties of deformation resistance and fracture resistance. Although the property of wear resistance of PEEK itself is not so sufficient, the property is intended to be improved by compositing PEEK with a carbon fiber. However, use of rigid carbon fiber may damage the femoral head component to be combined therewith, and thus a PEEK material having sufficient properties for the artificial joints has not been obtained.

Alternatively, it has also been studied to improve the property of slidability of the surface of the sliding portion by forming a coating layer on the surface of PE. For example, it is known a method for fixing a coating layer of a random copolymer comprising an allylamine and a group analogous to phosphorylcholine groups to the surface of a medical appliance, which is required to have an excellent sliding property such as an artificial joint, thereby providing a biocompatibility and a surface lubricity thereto (Patent Document 1).

When a polymerizable monomer having phosphorylcholine groups, for example, 2-methacryloyloxyethyl phosphorylcholine (MPC) is graft polymerized on the sliding surface of the artificial joint, which is formed by PE, an artificial joint component made from a polymer material which can suppress the production of a wear debris rather than those used in the prior art and which shows superior effect for suppressing the wear of the artificial joint (Patent Document 2).

MPC polymer produced by the method for Patent Document 2 is useful as the material for forming an ideal biocompatible surface. However, when the product resulted therefrom is used as a biocompatible material, it is desirable that no polymerization initiator remains on the surface of the substrate and in the graft polymer layer after performing the graft polymerization reaction. Therefore, according to the method for Patent Document 2, there was another problem that the radical initiator remaining after performing the graft polymerization reaction should be removed from the surface of the substrate and the graft polymer layer.

The method for Patent Document 2 is shown by the following Scheme 1:

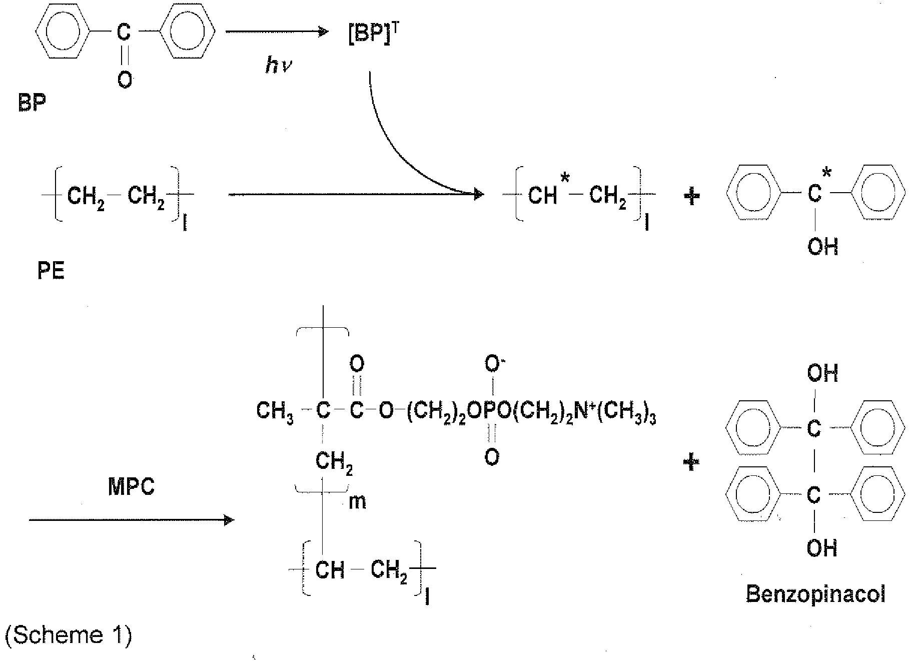

Patent Document 3 discloses a method for graft polymerization not using a polymerization initiator, but using a radiation such as gamma ray, wherein a sufficient grafting density could not be obtained in the graft polymerization due to generating radicals from both of the substrate and the monomer. In addition, there is a problem that the substrate could be degraded or embirttled by the above method, since the radiation such as gamma ray can penetrate to inside the substrate, thereby unnecessary of undesired breakage of molecular chains could occur inside the substrate.

### Prior Art Literatures

Patent Document 1: WO 01/05855 A1
Patent Document 2: JP 2003-310649 A
Patent Document 3: JP 2008-53041 A

EP1987849 discloses sliding material comprising an UHMPE substrate with a coating of a polymer containing phosphorylcholine groups.

### DISCLOSURE OF THE INVENTION

### PROBLEMS TO BE SOLVED BY THE INVENTION

Therefore, an object of the present invention is to dissolve the above problems. That is, the object of the present invention is to provide a polymer sliding material having an excellent durability together with a high lubricity and a biocompatibility and a method for producing such a polymer sliding material, and to provide an artificial joint member which comprises such a polymer sliding material and an artificial joint having an anti-dislocation function which comprises the above polymer sliding member.

### MEANS FOR SOLVING THE PROBLEMS

As a result of intensive studies to dissolve the above problems, the present inventors surprisingly discovered the following matters:
i) A friction coefficient of a coating layer (B) drastically decreases when the density of a graft layer exceeds a predetermined value in a polymer sliding material comprising a polymer substrate (A) having ketone groups on a surface and a coating layer (B) which coats at least a portion of the surface of the polymer substrate (A), wherein the coating layer (B) comprises (or consists of) a graft polymer layer containing phosphorylcholine groups;
ii) The above phenomenon corresponds to a drastic decrease of the friction coefficient of the coating layer (B) when a static contact angle with water on the coating layer (B) falls below a predetermined value, which angle is an indication of the hydrophilicity of the coating layer (B); and
iii) The degree of decrease of the frictional coefficient is related to the degree of the surface roughness of the polymer substrate (A) and the frictional coefficient and the static contact angle drastically decrease when an average surface roughness (Ra value) of the polymer substrate (A) falls below a predetermined value.

Then, the present invention has been accomplished.

That is, the present invention relates to a polymer sliding material comprising a polymer substrate (A) having ketone groups on a surface and a coating layer (B) which coats at least a portion of the surface of the polymer substrate (A), wherein the coating layer (B) comprises a graft polymer layer containing phosphorylcholine groups and having a density of at least 1.4 g/cm³, wherein the polymer substrate (A) has an average surface roughness (Ra) of not more than 0.02 µm.

The present invention also relates to the above polymer sliding material comprising a polymer substrate (A) having ketone groups on a surface and a coating layer (B) which coats at least a portion of the surface of the polymer substrate (A), wherein the coating layer (B) comprises a graft polymer layer containing phosphorylcholine groups and having a layer density of at least 1.4 g/cm3, wherein the polymer substrate (A) has an average surface roughness (Ra) of not more than 0.02 µm, and having a static contact angle with water of not more than 15 degrees.

The present invention also relates to an artificial joint member comprising the polymer sliding material obtained from the above-mentioned present invention.

The present invention also relates to an artificial joint which comprises the above-mentioned artificial joint member.

The present invention also relates to a method for producing a polymer sliding material comprising a polymer substrate (A) having ketone groups on a surface and a coating layer (B) which coats at least a portion of the surface of the polymer substrate (A), wherein the coating layer (B) comprises a graft polymer layer containing phosphorylcholine groups, comprising the steps of,
i) immersing the polymer substrate (A) in a reaction system containing a monomer (C) containing phosphorylcholine groups, and
ii) irradiating the polymer substrate (A) with light, thereby graft polymerizing the monomer (C) from the surface of the polymer substrate (A).

### EFFECTS OF THE INVENTION

The present invention provides a polymer sliding material comprising a polymer substrate (A) having ketone groups on a surface and having an average surface roughness (Ra) of not more than 0.02 µm, and a coating layer (B) which coats at least a portion of the surface of the polymer substrate (A), wherein the coating layer (B) comprises a graft polymer layer containing phosphoryicholine groups that has a biocompatibility and a hydrophilicity and having a high density of at least 1.4 g/cm³. Therefore, the polymer sliding material of the present invention shows a high lubricity which has not been conventionally attained, and additionally has an excellent durability, by which the wear resistance can be maintained over a long period of time. Since the coating layer (B) in the present invention has a highly hydrophilic surface having a static contact angle with water of not more than 15 degrees, and provides a sliding material showing a remarkable decrease of the friction coefficient compared with the conventional polymer sliding materials and having an excellent wear resistance. In the present invention, the coating layer (B) shows a remarkable reduction of the friction coefficient when the surface roughness of the polymer substrate (A) has a smoothness represented by an average surface roughness of not more than 0.02 µm. The present invention also provides a polymer sliding member comprising such a sliding material and an artificial joint comprising such a polymer sliding member. The artificial joint of the present invention can provide a polymer sliding member comprising a polymer substrate having relatively smaller thickness and having an excellent durability by attaining a sufficient wear resistance, load bearing properties and fracture resistance, even when the thickness of the polymer substrate is set comparatively thin.

### BRIEF DESCRIPTION OF THE DRAWINGS

Fig. 1 shows a relationship between the density (Graft Layer Density) and the friction coefficient (Friction Coefficient) of the graft layer (Examples 1 to 5, and Comparative Examples 1 to 6).
Fig. 2 shows a relationship between the static contact angle of water (Contact Angle) and the friction coefficient (Friction Coefficient) (Examples 1 to 5, and Comparative Examples 1 to 6).
Fig. 3 shows a relationship between the surface roughness (Average Surface Roughness: Ra) and the friction coefficient (Friction Coefficient) for PEEK of the polymer substrate (A).
Fig. 4 is a schematic illustration of an example where the construction of Example 1 is applied to an artificial hip joint.
Fig. 5 is a schematic illustration showing a motion range of an artificial hip joint where the construction of Example 1 is applied to an artificial hip joint.

### MODES FOR CARRYING OUT THE INVENTION

Hereafter, some modes for carrying out the present invention are illustrated more in detail.

In one of embodiments, the polymer sliding material of the present invention comprises a polymer substrate (A) having ketone groups on a surface and a coating layer (B) which coats at least a portion of the surface of the polymer substrate (A), wherein the coating layer (B) comprises (or consists of) a graft polymer layer containing phosphorylcholine groups and having a density of at least 1.4 g/cm3, wherein the polymer substrate (A) has an average surface roughness (Ra) of not more than 0.02 µm.

When the density of the layer containing the phosphorylcholine groups exceeds 1.4 g/cm³, an effect of drastic decrease in the friction coefficient of the coating layer (B) is achieved. The density of the layer containing the phosphorylcholine groups is preferably at least 1.5 g/cm³, and more preferably at least 2.0 g/cm³

In the graft polymerization in the present invention, the backbone (trunk) polymer is the polymer substrate (A) and the graft (branch) is the polymer containing the phosphorylcholine groups.

In another embodiment, the polymer sliding material of the present invention comprises a polymer substrate (A) having ketone groups on a surface and a coating layer (B) which coats at least a portion of the surface of the polymer substrate (A), wherein the coating layer (B) comprises a graft polymer layer containing phosphorylcholine groups and having a layer density of at least 1.4 g/cm3, wherein the polymer substrate (A) has an average surface roughness (Ra) of not more than 0.02 µm, and having a static contact angle with water of not more than 15 degrees.

When the static contact angle falls below 15 degrees, an effect of drastic decrease in the friction coefficient of the coating layer (B) is achieved. The static contact angle is preferably not more than 10 degrees, and more preferably not more than 8 degrees.

In further embodiment, the polymer sliding material of the present invention preferably comprises a polymer substrate (A) having ketone groups on a surface and a coating layer (B) which coats at least a portion of the surface of the polymer substrate (A), wherein the coating layer (B) comprises a graft polymer layer containing phosphorylcholine groups and having a density of at least 1.4 g/cm³, wherein the polymer substrate (A) has an average surface roughness (Ra) of not more than 0.02 µm, and the coating layer (B) has a static contact angle with water of not more than 15 degrees.

The polymer substrate (A) of the present invention has an average surface roughness (Ra) of not more than 0.02 µm, preferably not more than 0.015 µm, and particularly preferably not more than 0.01 µm. When the average surface roughness (Ra) is not more than 0.02 µm, the effects of the decrease in the friction coefficient and the improvement of the hydrophilicity become remarkable.

Especially, the polymer sliding material of the present invention preferably comprises a polymer substrate (A) having ketone groups on a surface and a coating layer (B) which coats at least a portion of the surface of the polymer substrate (A), wherein the coating layer (B) comprises a graft polymer layer containing phosphorylcholine groups and having a density of at least 1.4 g/cm³, and has a static contact angle with water of not more than 15 degrees, and the polymer substrate (A) to be graft polymerized has an average surface roughness (Ra) of not more than 0.02 µm.

The polymer substrate (A) of the present invention is preferably a polymer substrate containing an aromatic ketone. Specifically, the polymer substrate (A) may comprise any of:
a polymer selected from the group consisting of polyether ketone (PEK), polyether ether ketone (PEEK), polyether ketone ketone (PEKK), polyether ether ketone ketone (PEEKK), polyether ketone ether ketone ketone (PEKEKK) and poly aryl ether ketone (PAEK);
a composite polymer made of at least two types of polymers selected from the above polymers, and
a fiber-reinforced polymer comprising a matrix of any polymer selected from the above polymers and the above composite polymers. The polymer substrate (A) serves as the trunk polymer in the graft polymerization according to the present invention.

The graft polymer layer containing the phosphorylcholine groups of the present invention has a constituent unit derived from a monomer which contains the phosphorylcholine groups, which includes 2-methacryloyloxyethyl phosphorylcholine (MPC), 2-acryloyloxyethyl phosphorylcholine, 4-methacryloyloxybutyl phosphorylcholine, 6-methacryloyloxyhexyl phosphorylcholine, omega-methacryloyloxyethylene phosphorylcholine and 4-styryloxybutyl phosphorylcholine. Among all, the constituent unit derived from a (meth)acrylate monomer having the phosphorylcholine group is preferable, and 2-methacryloyloxyethylphosphorylcholine is particularly preferable. The phosphorylcholine group is a phospholipid as a constituent of a biomembrane, and is excellent in biocompatibility as well as in the hydrophilicity and the lubricity, so that it is preferable to use the polymer sliding material of the present invention which has the coating layer (B) formed by MPC. The polymer containing the phosphorylcholine groups serves as the branch (graft) polymer for the graft polymerization in the present invention.

Preferably, the graft polymer layer is formed by a surface graft polymerization resulted from a photo-irradiation without containing a polymerization initiator. Specifically, the surface graft polymerization is preferably resulted by immersing the polymer substrate (A) in a reaction system comprising a monomer (C) containing the phosphorylcholine groups, irradiating the polymer substrate (A) with light and graft polymerizing the monomer from the surface of the polymer substrate (A). The wavelength of the light being irradiated is usually in the range from 10 to 400 nm.

The present invention provides an artificial joint member comprising the polymer sliding material obtained by the above-mentioned present invention. Particularly, the artificial joint member is preferable, in which the polymer substrate (A) forms a base of the artificial joint member, and the coating layer (B) is formed on at least a sliding face of the artificial joint member. Furthermore, the artificial joint member is preferable, in which a ball head to be combined has a diameter of at least 32 mm and a polymer substrate has a thickness in the range from 3 to 6mm in the present invention. The present invention provides an artificial joint comprising the artificial joint member.

The present invention provides a method for producing the polymer sliding material comprising the polymer substrate (A) having ketone groups on a surface and a coating layer (B) which coats at least a portion of the surface of the polymer substrate (A), wherein the coating layer (B) comprises a graft polymer layer containing phosphorylcholine groups and having a layer density of at least 1.4 g/cm3, wherein the polymer substrate (A) has an average surface roughness (Ra) of not more than 0.02 µm. The method for producing the polymer sliding material comprises the steps of:
i) immersing the polymer substrate (A) in a reaction system containing a monomer (C) containing phosphorylcholine groups in a monomer concentration range from 0.1 to 2.0 mol/L, and
ii) irradiating the polymer substrate (A) with light having the wavelength from 10 to 400 nm at an intensity in the range from 1.0 to 10.0 mW/cm², thereby graft polymerizing the monomer (C) from the surface of the polymer substrate (A).

In the method for producing the polymer sliding material, the wavelength of the light irradiated may be in the range from 10 to 400 nm, preferably in the range from 200 to 400 nm, and more preferably in the range from 300 to 400 nm.

In the above-mentioned production method, the concentration of the monomer (C) may be preferably in the range from 0.25 to 1.0 mol/L, and more preferably in the range from 0.25 to 0.50 mol/L.

In the above-mentioned production method, the intensity of the light may be preferably in the range from 1.5 to 8.0 mW/cm², and more preferably in the range from 4.0 to 6.0 mW/cm².

In the above-mentioned production method, the graft polymerization is preferably carried out without using a polymerization initiator. Since the polymerization initiator is not contained, the obtained polymer sliding material can avoid the problems including such as the degradation of the polymer sliding material caused by the residue of the polymerization initiator.

In the above-mentioned production method, the temperature of the graft polymerization performed by irradiating light may be in the range from 10 to 90°C, preferably in the range from 20 to 80°C, and more preferably in the range from 50 to 70°C.

In the above-mentioned production method, the graft polymerization time performed by irradiating light may be usually in the range from 20 minutes to 10 hours, preferably from 30 minutes to 5 hours, and more preferably from 45 minutes to 90 minutes, depending on the other conditions including the polymerization temperature.

In the above-mentioned production method, a suitable solvent includes water, alcohols and an aqueous solution of the alcohols. The solvent to be used needs to at least satisfy the requirements consisting of dissolving or dispersing the monomer, being incapable of eroding or dissolving the substrate and being incapable of adversely affecting the graft polymerization of the present invention.

In the present invention, the schematic diagram of the graft reaction, wherein PEEK is used as the substrate and 2-methacryloyloxyethyl phosphorylcholine (MPC) is used as the compound containing the phosphorylcholine groups, is represented as follows:

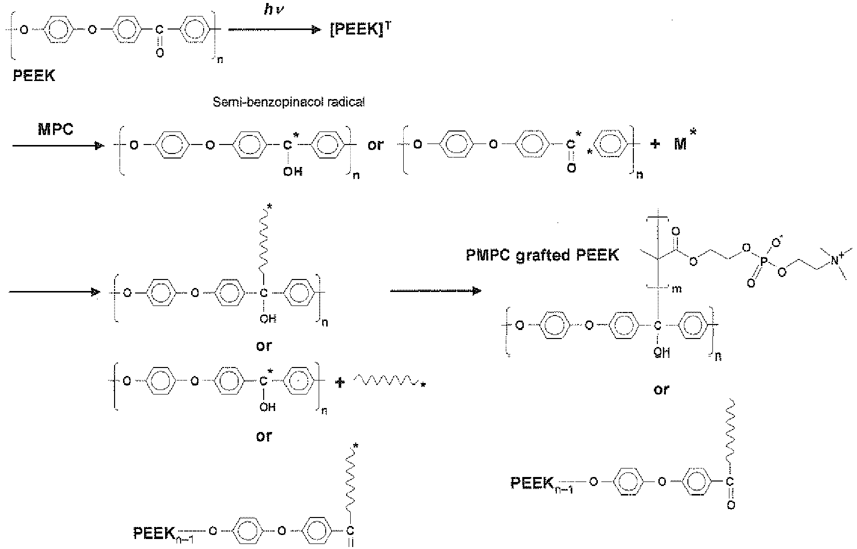

In the above-mentioned production method, the polymer substrate (A) has an average surface roughness (Ra) of not more than 0.02 µm, more preferably not more than 0.015 µm, and particularly preferably not more than 0.01 µm. When the polymer substrate (A) has the surface roughness (Ra) of not more than 0.02 µm, the effects of the decrease in the friction coefficient and the improvement of the hydrophilicity become remarkable.

The graft polymer layer of the present invention is preferably formed by the surface graft polymerization resulted from a photo-irradiation without containing a polymerization initiator. Since the polymerization initiator is not contained, the problems such as the degradation of the polymer sliding material, 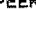 which could be caused by the residue of the polymerization initiator, can be avoided.

### EXAMPLES

Hereinafter, the present invention will be more specifically illustrated with reference to Examples, but the present invention is not limited thereto.

### [Identification of the graft polymerization]

A graft polymerization of a MPC monomer to a surface of a PEEK polymer substrate was confirmed by
i) the presence of an IR absorption peak (1060 cm⁻¹, 1720 cm⁻¹) resulted from MPC according to FT-IR analysis (Model FT/IR 615, manufactured by JASCO Corp.), and
ii) the presence of a peak (P-O bond) resulted from MPC according to XPS analysis (Model AXIS-HSi 165, manufactured by KRATOS ANALYTICAL).

### [Measurement of thickness of MPC polymer film obtained by graft polymerization]

The thickness of a MPC polymer film obtained by a graft polymerization was measured by the following procedure:
A sample to be used for the measurement was embedded in an epoxy resin, stained with ruthenium tetrachloride and ultrathin sections were sliced therefrom using an ultramicrotome. The observation was performed with using a transmission electron microscope (TEM) (Model JEM-1010 manufactured by JEOL) at an acceleration voltage of 100 kV.

### [Analysis of density of graft layer]

The density of a coating layer (B) was obtained by evaluating the increase of weight (g) after a graft polymerization of a monomer containing phosphorylcholine groups on a surface of a polymer substrate (A) as a weight of the coating layer (B), and dividing the above weight increase value by the volume (cm³) of the coating layer (B) obtained from the thickness of the coating layer (B) measured according to the above and a surface area of the polymer substrate (A). The weight increase was measured by a precision balance (Sartorius Supermicro S4, manufactured by Sartorius AG).

### [Measurement of static contact angle]

A static contact angle was measured by a surface contact angle goniometer (model DM300, manufactured by Kyowa Interface Science Co., Ltd.) using a sessile drop method according to ISO Standard 15989. That is, purified water having a droplet amount of 1 µL was deposited on a surface of the sample and the contact angle was measured at 60 seconds after deposition. The obtained value was taken as "the static contact angle with water."

### [Measurement of the friction coefficient]

A friction coefficient of a surface of a coating layer (B) was measured by a Pin-on-plate type friction tester (Tribostation Type 32, manufactured by Shinto Scientific Co., Ltd.) under conditions including a load of 0.98N, a slide velocity of 50 mm/sec, a slide distance of 25 mm, a bounce frequency of 1 Hz, a room temperature and a water lubrication environment.

### [Measurement of surface roughness]

As for a surface roughness of a polymer substrate (A), an average surface roughness (Ra: µm) was measured by a stylus type surface roughness meter (Surftest SV-3100, manufactured by Mitsutoyo Corporation) according to JIS B 0601.

### Example 1 (comparative example, not forming part of the invention)

10 PEEK was used as the substrate, MPC was used as the monomer and water was used as the solvent for dispersing the monomer.

First, a PEEK sample (length 10 cm x width 1 cm x thickness 0.3 cm; weight 3.9 g: trade name 450G, manufactured by VICTREX) to be used as the substrate was ultrasonically cleaned in ethanol, thereby the surface was cleaned. Separately, a 0.5 mol/L aqueous solution of MPC was prepared. The above-prepared aqueous MPC solution in an amount of 15 mL was introduced into a quartz glass vessel.

While maintaining the temperature of the above aqueous MPC solution at 60°C, the PEEK sample was immersed in the aqueous MPC solution. The PEEK sample was irradiated with ultraviolet rays having a wavelength of 300 to 400 nm for 90 minutes, thereby performed a graft polymerization reaction. After irradiating with ultraviolet rays, the PEEK sample was picked up from the aqueous MPC solution, sufficiently rinsed with pure water and dried to obtain a polymer sliding material.

### Example 2 (comparative example, not forming part of the invention)

The same procedure was performed as in Example 1, except that the irradiation time of the ultraviolet rays was changed to 45 minutes, and thereby a polymer sliding material was obtained.

### Example 3 (comparative example, not forming part of the invention)

The same procedure was performed as in Example 1, except that the irradiation time of the ultraviolet rays was changed to 23 minutes, and thereby a polymer sliding material was obtained.

### Example 4 (comparative example, not forming part of the invention)

The same procedure was performed as in Example 1, except that the concentration of MPC was changed to 0.25 mol/L, and thereby a polymer sliding material was obtained.

### Example 5 (comparative example, not forming part of the invention)

The same procedure was performed as in Example 1, except that the concentration of MPC was changed to 0.25 mol/L and the irradiation time of the ultraviolet rays was changed to 45 minutes, and thereby a polymer sliding material was obtained.

### Comparative Example 1 (comparative example, not forming part of the invention)

25 The same procedure was performed as in Example 1, except that the irradiation time of the ultraviolet rays was changed to 10 minutes, and thereby a polymer sliding material was obtained.

### Comparative Example 2 (comparative example, not forming part of the invention)

5 The same procedure was performed as in Example 1, except that the irradiation time of the ultraviolet rays was changed to 5 minutes, and thereby a polymer sliding material was obtained.

### Comparative Example 3 (comparative example, not forming part of the invention)

10 The same procedure was performed as in Example 4, except that the concentration of MPC was changed to 0.25 mol/L and the irradiation time of the ultraviolet rays was changed to 23 minutes, and thereby a polymer sliding material was obtained.

### Comparative Example 4 (comparative example, not forming part of the invention)

The same procedure was performed as in Example 4, except that the concentration of MPC was changed to 0.25 mol/L and the irradiation time of the ultraviolet rays was changed to 10 minutes, and thereby a polymer sliding material was obtained.

### Comparative Example 5 (comparative example, not forming part of the invention)

The same procedure was performed as in Example 4, except that the concentration of MPC was changed to 0.25 mol/L and the irradiation time of the ultraviolet rays was changed to 5 minutes, and thereby a polymer sliding material was obtained.

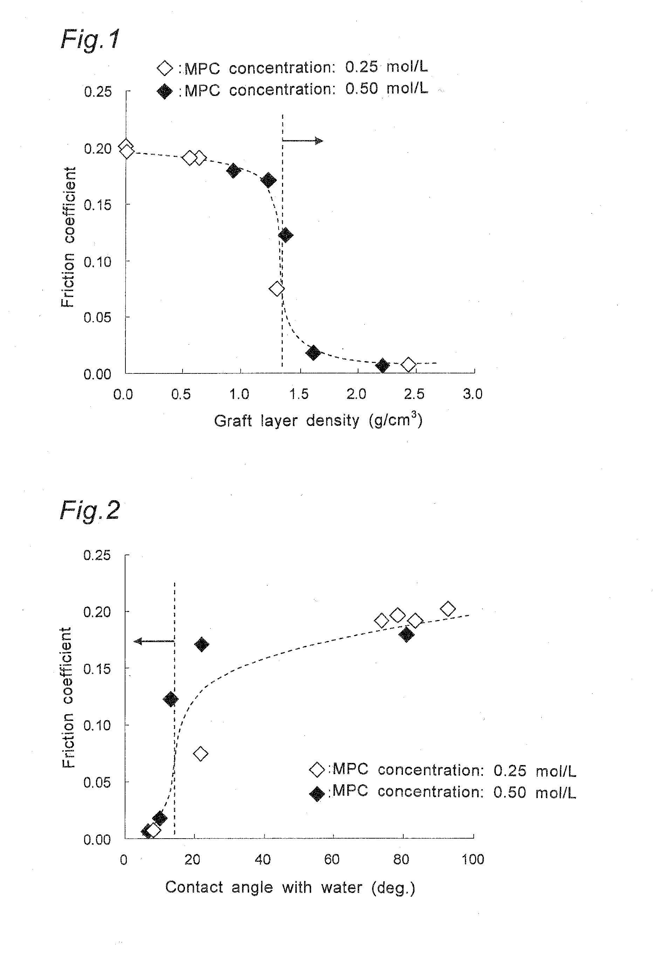

Each of a graft density, a contact angle and a friction coefficient was measured for each of the polymer sliding materials obtained from Examples 1 to 5 and Comparative Examples 1 to 5. The obtained results are shown in Fig. 1 (Relationship between graft layer density and friction coefficient) and Fig. 2 (Relationship between static contact angle of water and friction coefficient). The results reveal that the friction coefficient drastically decreases along with the increase of the density of the graft layer at a boundary of the graft layer density of 1.4 g/cm³ (Fig. 1), and that the friction coefficient drastically decreases along with the decrease of the contact angle at a boundary of the contact angle of 15 degrees (Fig. 2).

### Example 6

The same procedure was performed as in Example 1, except that PEEK having the surface roughness (Ra) less than 0.01 µm (Ra<0.01 µm) was used as the polymer substrate, and thereby a polymer sliding material was obtained.

### Comparative Example 6

The same procedure was performed as in Example 1, except that PEEK having the surface roughness (Ra) less than 2 µm (Ra<2 µm) was used as the polymer substrate, and thereby a polymer sliding material was obtained.

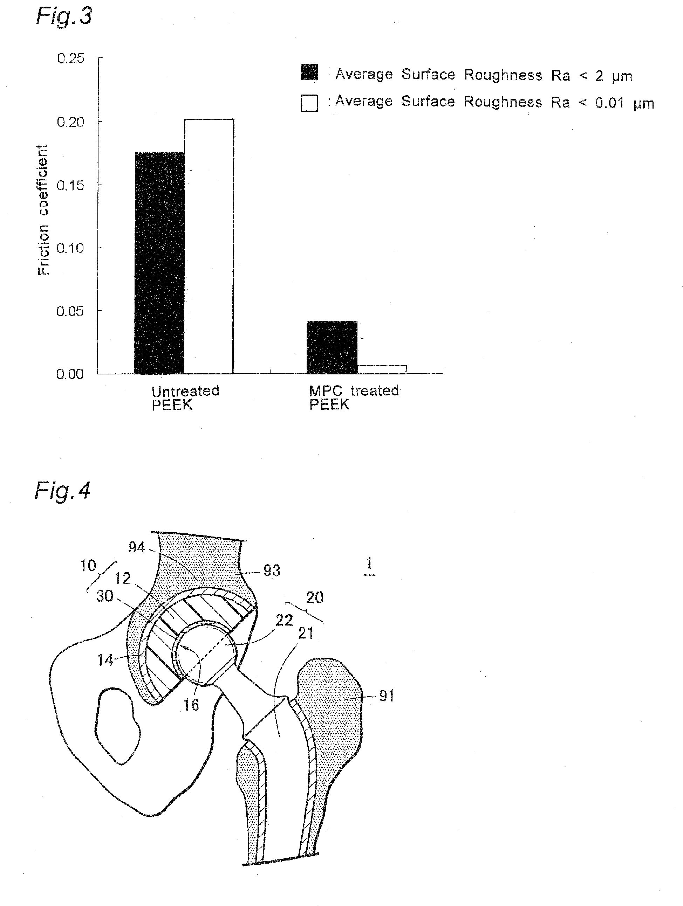

A friction coefficient of each of the polymer sliding materials obtained from Example 6 and Comparative Example 6 was measured, and each value was compared with the values of the friction coefficient before performing the graft treatment. The results shown in Fig. 3 reveal that the decreasing effect of the friction coefficient by the graft treatment with the MPC polymer is remarkable in PEEK which has smaller value of the surface roughness.

Fig. 4 is a schematic diagram of an artificial hip joint 1 as an example of the artificial joint formed by using the artificial joint member of the present invention. The artificial hip joint 1 is constituted from a sliding member (an acetabular cup) 10 that is fixed onto an acetabulum 94 of a pelvis 93 and a femoral stem 20 that is fixed onto a proximal end of a femur 91. The acetabular cup 10 comprises a cup substrate 12 that has a substantially semi-spherical acetabulum fixing surface 14 and a sliding surface 16 of concave semi-spherical shape, and a coating layer 30 with which the sliding surface 16 is covered. A ball head 22 of the femoral stem 20 is slidably fitted to the sliding surface 16 of the **a**cetabular cup 10, so as to function as the hip joint.

According to Example 1, (comparative example, not forming part of the invention) a coating layer 30 made of MPC, which serves as the sliding surface 16 of the cup substrate 12, was formed by the graft polymerization using PEEK as the cup substrate 12 and MPC as the monomer. In this artificial hip joint, excellent slidability and movability (anti-disiocation function) were attained, even when the thickness of the coating layer 30 was preferably from 50 to 200 nm, the thickness of the cup substrate 12 was preferably from 3 to 6 mm, and the diameter of the (femoral) ball head 22 was preferably from 40 to 48 mm.

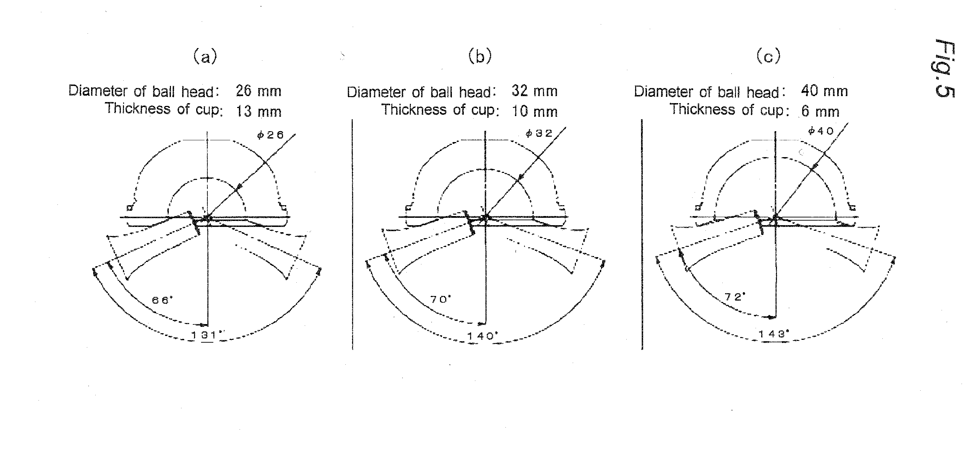

Fig. 5 shows a schematic illustration of the range of motion of the artificial joint member of the present invention. Fig. 5(a) shows an example where a diameter of 26 mm was adopted as the ball head which is the maximum size for corresponding to the thickness of 13 mm of the cup substrate. Fig. 5(b) shows an example where a diameter of 32 mm was adopted as the ball head which is the maximum size for corresponding to the thickness of 10 mm of the cup substrate. Fig. 5(c) shows an example where a diameter of 40 mm was adopted as the ball head which is the maximum size for corresponding to the thickness of 6 mm of the cup substrate.

It was confirmed that, in the order of Fig. 5(a), Fig. 5(b) and Fig. 5(c), the thickness of the cup substrate decreased and the diameter of the ball head (the femoral head) increased, and the range of motion of the artificial joint member correspondingly increased. Specifically, in the example of Fig. 5(a) which corresponds to the conventional artificial joint having a thickness of 13 mm of the cup substrate and a diameter of 26 mm of the femoral head, the range of motion was 131 degrees. In the case of Fig. 5(b), which is an example of the present invention, the thickness of 10 mm of the cup substrate and the diameter of 32 mm of the femoral head could be attained, and the range of motion was 140 degrees. In the case of Fig. 5(c), which is another example of the present invention, the thickness of 6 mm of the cup substrate and the diameter of 40 mm of the femoral head could be attained, and the range of motion was 143 degrees. The increase of the range of motion of the artificial joint means that the artificial joint has a structure wherein a dislocation is hardly caused. The thickness of the cup substrate was conventionally required to be 10 mm or more in view of a limitation from strength, since PE was used as the substrate. However, using a polymer having higher strength such as PEEK as in the present invention, a cup having a thickness of 3 to 6 mm may be designed and a sufficient safety relating to the strength can also be secured.

From the above, it was confirmed that the monomers having phosphorylcholine groups and vinyl groups such as acrylates may be graft polymerized onto the surface of the polymer substrate having ketone groups on the surface by irradiating with light, and the surface of the resultant artificial joint member has a low contact angle with water and a low coefficient of dynamic friction. In addition, it was also confirmed that the artificial joint produced using those artificial joint members could have a structure having a wide range of motion. Therefore, the artificial joint simultaneously having high lubricity, biocompatibility and anti-dislocation function can be provided by applying the polymer sliding member of the present invention.

### INDUSTRIAL APPLICABILITY

The polymer sliding material of the present invention is suitable to apply to an artificial joint member. The artificial joint member of the present invention is suitable to apply to an artificial joint. The acetabular cup which was produced using the artificial joint member of the present invention can practically use the femoral head having a size which was not conventionally considered to be adequate, since the thickness of the acetabular cup per se can be made thin. Therefore, the artificial joint having the enlarged range of motion and being useful for prevention of dislocation may be provided, which is valuable for a medical industry.

## Claims

1. A polymer sliding material comprising a polymer substrate (A) having ketone groups on a surface and a coating layer (B) which coats at least a portion of the surface of the polymer substrate (A), wherein the coating layer (B) comprises a graft polymer layer containing phosphorylcholine groups and having a layer density of at least 1.4 g/cm³, wherein the polymer substrate (A) has an average surface roughness (Ra) of not more than 0.02 µm.

2. The polymer sliding material according to claim 1, wherein the coating layer (B) has a static contact angle with water of not more than 15 degrees.

3. The polymer sliding material according to any one of claims 1 and 2, wherein the coating layer (B) has a thickness from 10 to 200 µm.

4. The polymer sliding material according to any one of claims 1 to 3, wherein the polymer substrate (A) is a polymer substrate containing aromatic ketones.

5. The polymer sliding material according to claim 4, wherein the polymer substrate (A) comprises:
a polymer selected from the group consisting of polyether ketone (PEK), polyether ether ketone (PEEK), polyether ketone ketone (PEKK), polyether ether ketone ketone (PEEKK), polyether ketone ether ketone ketone (PEKEKK) and poly aryl ether ketone (PAEK),
a composite polymer made of at least two types of polymers selected from the above polymers, and
a fiber-reinforced polymer comprising a matrix of any polymer selected from the above polymer and the above composite polymer.

6. The polymer sliding material according to any one of claims 1 to 5, wherein the graft polymer layer containing the phosphorylcholine groups has a constituent unit derived from a (meth)acrylate polymer having the phosphorylcholine groups.

7. The polymer sliding material according to claim 6, wherein the (meth)acrylate polymer containing the phosphorylcholine groups is 2-methacryloyloxyethyl phosphorylcholine (MPC).

8. The polymer sliding material according to any one of claims 1 to wherein the graft polymer layer is formed by a surface graft polymerization resulted from a photo-irradiation without containing a polymerization initiator.

9. The polymer sliding material according to claim 8, wherein the surface graft polymerization is conducted by immersing the polymer substrate (A) in a reaction system containing a monomer (C) containing the phosphorylcholine groups, irradiating the polymer substrate (A) with light and graft polymerizing the monomer from the surface of the polymer substrate (A).

10. The polymer sliding material according to claim 8 or 9, wherein the light to be irradiated has the wavelength from 10 to 400 nm.

11. An artificial joint member comprising the polymer sliding material according to any one of claims 1 to 10.

12. A method for producing a polymer sliding material as defined in any one of the claims 1 to 10, wherein the method comprises the steps of,
i) immersing the polymer substrate (A) in a reaction system containing a monomer (C) containing phosphorylcholine groups in a monomer concentration range from 0.1 to 2.00 mol/L, and
ii) irradiating the polymer substrate (A) with light having the wavelength from 10 to 400 nm at an intensity in the range from 1.0 to 10.0 mW/cm², to graft polymerize the monomer (C) from the surface of the polymer substrate (A).

## Patentansprüche

1. Polymergleitmaterial, umfassend ein Polymersubstrat (A) mit Ketongruppen auf einer Oberfläche und eine Beschichtungsschicht (B), die zumindest einen Bereich der Oberfläche des Polymersubstrates (A) beschichtet, worin die Beschichtungsschicht (B) eine Pfropfpolymerschicht enthält, umfassend Phosphorylcholingruppen und mit einer Schichtdichte von wenigstens 1,4 g/cm³, worin das Polymersubstrat (A) eine durchschnittliche Oberflächenrauigkeit (Ra) von nicht mehr als 0,02 µm hat.

2. Polymergleitmaterial gemäß Anspruch 1, worin die Beschichtungsschicht (B) einen statischen Kontaktwinkel mit Wasser von nicht mehr als 15 Grad hat.

3. Polymergleitmaterial gemäß einem der Ansprüche 1 und 2, worin die Beschichtungsschicht (B) eine Dicke von 10 bis 200 µm hat.

4. Polymergleitmaterial gemäß einem der Ansprüche 1 bis 3, worin das Polymersubstrat ein Polymersubstrat ist, das aromatische Ketone enthält.

5. Polymergleitmaterial gemäß Anspruch 4, worin das Polymersubstrat (A) enthält:
ein Polymer, ausgewählt aus der Gruppe bestehend aus Polyetherketon (PEK), Polyetheretherketon (PEEK), Polyetherketonketon (PEKK), Polyetheretherketonketon (PEEKK), Polyetherketonetherketonketon (PEKEKK) und Polyaryletherketon,
ein Verbundpolymer, erzeugt aus zumindest zwei Typen von Polymeren, ausgewählt aus den obigen Polymeren, und
ein faserverstärktes Polymer, umfassend eine Matrix von irgendeinem Polymer, ausgewählt aus dem obigen Polymer und dem obigen Verbundpolymer.

6. Polymergleitmaterial gemäß einem der Ansprüche 1 bis 5, worin die Pfropfpolymerschicht, die die Phosphorylcholingruppen enthält, eine Bestandteilseinheit enthält, die von einem (Meth)acrylatpolymer mit den Phosphorylcholingruppen abgeleitet ist.

7. Polymergleitmaterial gemäß Anspruch 6, worin das (Meth)acrylatpolymer mit den Phosphorylcholingruppen 2-Methacryloyloxyethylphosphorylcholin (MPC) ist.

8. Polymergleitmaterial gemäß einem der Ansprüche 1 bis 7, worin die Pfropfpolymerschicht durch eine Oberflächenpfropfpolymerisation gebildet ist, die von einer Fotobestrahlung ohne Vorhandensein eines Polymerisationsinitiators resultiert.

9. Polymergleitmaterial gemäß Anspruch 8, worin die Oberflächenpfropfpolymerisation durchgeführt wird durch Eintauchen des Polymersubstrates (A) in ein Reaktionssystem, umfassend ein Monomer (C), umfassend die Phosphorylcholingruppen, Bestrahlen des Polymersubstrates (A) mit Licht und Pfropfpolymerisation des Monomers von der Oberfläche des Polymersubstrates (A) .

10. Polymergleitmaterial gemäß Anspruch 8 oder 9, worin das zu bestrahlende Licht eine Wellenlänge von 10 bis 400 nm hat.

11. Künstliches Gelenkteil, umfassend das Polymergleitmaterial gemäß einem der Ansprüche 1 bis 10.

12. Verfahren zur Erzeugung eines Polymergleitmaterials wie in einem der Ansprüche 1 bis 10 definiert, worin das Verfahren die folgenden Schritte umfasst:
i) Eintauchen des Polymersubstrates (A) in ein Reaktionssystem, umfassend ein Monomer (C), umfassend Phosphorylcholingruppen in einem Bereich in einer Monomerkonzentration von 0,1 bis 2,00 mol/l und
ii) Bestrahlen des Polymersubstrates (A) mit Licht mit der Wellenlänge von 10 bis 400 nm bei einer Intensität im Bereich von 1,0 bis 10,0 mW/cm², zum Pfropfpolymerisieren des Monomers (C) von der Oberfläche des Polymersubstrates (A).

## Revendications

1. Matériau coulissant en polymère comprenant un substrat en polymère (A) ayant des groupes cétone sur une surface et une couche de revêtement (B) qui revêt au moins une partie de la surface du substrat en polymère (A), dans lequel la couche de revêtement (B) comprend une couche de polymère greffé contenant des groupes phosphorylcholine et ayant une masse volumique de couche d'au moins 1,4 g/cm³, et dans lequel le substrat en polymère (A) a une rugosité de surface moyenne (Ra) ne dépassant pas 0,02 µm.

2. Matériau coulissant en polymère selon la revendication 1, dans lequel la couche de revêtement (B) a un angle de contact statique avec l'eau ne dépassant pas 15 degrés.

3. Matériau coulissant en polymère selon l'une quelconque des revendications 1 et 2, dans lequel la couche de revêtement (B) a une épaisseur de 10 à 200 µm.

4. Matériau coulissant en polymère selon l'une quelconque des revendications 1 à 3, dans lequel le substrat en polymère (A) est un substrat en polymère contenant des cétones aromatiques.

5. Matériau coulissant en polymère selon la revendication 4, dans lequel le substrat en polymère (A) comprend :
un polymère choisi dans le groupe constitué par une polyéther-cétone (PEK), une polyéther-éther-cétone (PEEK), une polyéther-cétone-cétone (PEKK), une polyéther-éther-cétone-cétone (PEEKK), une polyéther-cétone-éther-cétone-cétone (PEKEKK) et une polyaryl-éther-cétone (PAEK),
un polymère composite fait d'au moins deux types de polymères choisis parmi les polymères ci-dessus, et
un polymère renforcé par des fibres comprenant une matrice d'un polymère quelconque choisi parmi le polymère ci-dessus et le polymère composite ci-dessus.

6. Matériau coulissant en polymère selon l'une quelconque des revendications 1 à 5, dans lequel la couche de polymère greffé contenant les groupes phosphorylcholine a un motif constitutif dérivé d'un polymère de (méth)acrylate ayant les groupes phosphorylcholine.

7. Matériau coulissant en polymère selon la revendication 6, dans lequel le polymère de (méth)acrylate contenant les groupes phosphorylcholine est la 2-méthacryloyloxyéthyl-phosphorylcholine (MPC).

8. Matériau coulissant en polymère selon l'une quelconque des revendications 1 à 7, dans lequel la couche de polymère greffé est formée par une polymérisation par greffage en surface résultant d'une photo-irradiation en l'absence d'un amorceur de polymérisation.

9. Matériau coulissant en polymère selon la revendication 8, dans lequel la polymérisation par greffage en surface est effectuée par immersion du substrat en polymère (A) dans un système réactionnel contenant un monomère (C) contenant les groupes phosphorylcholine, irradiation du substrat polymère (A) avec une lumière, et polymérisation par greffage du monomère depuis la surface du substrat en polymère (A).

10. Matériau coulissant en polymère selon la revendication 8 ou 9, dans lequel la lumière devant être irradiée a une longueur d'onde de 10 à 400 nm.

11. Membre d'articulation artificielle comprenant le matériau coulissant en polymère selon l'une quelconque des revendications 1 à 10.

12. Procédé pour produire un matériau coulissant en polymère tel que défini dans l'une quelconque des revendications 1 à 10, lequel procédé comprend les étapes suivantes :
i) immersion du substrat en polymère (A) dans un système réactionnel contenant un monomère (C) contenant des groupes phosphorylcholine à une concentration du monomère située dans la plage allant de 0,1 à 2,00 mol/l, et
ii) irradiation du substrat en polymère (A) avec une lumière ayant une longueur d'onde de 10 à 400 nm à une intensité située dans la plage allant de 1,0 à 10,0 mW/cm², pour polymériser par greffage le monomère (C) depuis la surface du substrat en polymère (A).
